# EUROPEAN PATENT SPECIFICATION

(11) **EP 0 862 414 B1**
(45) Date of publication and mention of the grant of the patent: **05.12.2001**
(21) Application number: 96931025.9
(22) Date of filing: 05.09.1996
(51) Int. Cl.: A61K 9/00

(54) **OPHTHALMIC COMPOSITIONS CONTAINING CYCLODEXTRINS AND QUATERNARY AMMONIUM COMPOUNDS**
OPHTALMISCHE ZUSAMMENSETZUNGEN DIE CYCLODEXTRINEN UND QUATERNÄREN AMMONIUMVERBINDUNGEN ENTHALTEN
COMPOSITIONS OPHTALMIQUES CONTENANT DES CYCLODEXTRINES AINSI QUE DES COMPOSES D'AMMONIUM QUATERNAIRE

(30) Priority: 18.09.1995 EP 95810575
(43) Date of publication of application: 09.09.1998
(73) Proprietor: Novartis AG, 4056 Basel (CH); Novartis-Erfindungen Verwaltungsgesellschaft m.b.H., 1235 Wien (AT)
(72) Inventor: KIS, György, Lajos, CH-8273 Triboltingen (CH); FETZ, Andrea, CH-8006 Zürich (CH); SCHOCH, Christian, CH-4132 Muttenz (CH)
(86) International application number: EP9603898
(87) International publication number: WO9710805

(56) References cited:
- EP-A- 0 306 984
- EP-A- 0 349 487
- WO-A-94/12217
- WO-A-94/15582

## Description

The present invention describes a pharmaceutical composition, in particular a preserved ophthalmic composition, comprising a cyclodextrin, a quaternary ammonium salt, an alkylene glycol and a pharmaceutically active compound.

Numerous ophthalmic compositions have been described in prior art. The majority of the prior art ophthalmic compositions are dealing with those properties which characterize the human tears and which are e.g. the osmolality, the viscosity or the pH. Other publications related to such compositions are directed towards the preservation of ophthalmic compositions. The addressed topics can be approached with different solutions. A few of these proposed solutions are shortly reviewed infra.

EP 349 487 describes an antimicrobial ophthalmic solution comprising a quaternary ammonium salt, namely Bradosol ® , as the microbicide and methods of using the same.

Ophthalmic solutions containing hydroxyethyl cellulose are described in GB 2 169 508, which solutions may serve as artificial tears.

EP 76 136 describes an aqueous disinfecting solution for contact lenses, which solutions contain certain polymeric quaternary ammonium compounds, such as e.g. Onamer M™.

DE 2 839 752 (Toko) discloses an ophthalmological gel, which is a homogenous mixture of an aqueous solution of a carboxy vinyl polymer with a polymer content of 0.05 to 5 % by weight, a water-soluble basic compound, a therapeutically effective amount of a common ophthalmological drug, having a pH in the range of 5 to 8 and a viscosity of 1000 to 100'000 mPas at 20°C, characterized in that it comprises 0.001 to 0.5 % weight NaCI.

GB 2 196 265 (Resdevco) discloses eye drops consisting of an essentially isotonic solution of a physiologically acceptable organic humectant in combination with required adjuvants or auxiliaries, containing less than 1.5 millimol/liter inorganic salt.

EP 37 043 (Wellcome) describes an isotonic liquid pharmaceutical formulation comprising trimethoprim and polymyxin, a vehicle therefor, a mercury containing preservative and an isotonic agent, characterized in that the isotonic agent comprises a non-ionic polyhydroxy compound.

Cyclodextrins have been proposed in several applications which are dealing with ophthalmic compositions. WO 94/15582 (Javitt et al.) describes e.g. an ophthalmic composition comprising an effective amount of a carbonic anhydrase inhibitor and an amount of an amorphous (β-cyclodextrin effective in increasing the bioavailability of the carbonic anhydrase inhibitor when coadministered topically.

β- and γ-cyclodextrins, in particular partially etherified derivatives thereof, have been proposed in EP 149 197 and EP 197 571 (both to Janssen) for pharmaceutical preparations comprising inclusion complexes of medicinal substances which are sparingly soluble in water or instable in water.

It is also known to preserve eye medicaments by means of quaternary ammonium salts (see, for example, EP-A-306,984). Only a very few quaternary ammonium salts have found practical application for the preservation of eye medicaments - evidently because of the multifarious requirements which must be fulfilled - these are, in particular, the three substances mentioned explicitly in EP-A-306,984, cetyltrimethylammonium bromide, cetylpyridinium chloride and benzalkonium chloride (cf. also EP-A-242,328). A preferred preservative for eye medicaments at the present time is typically benzalkonium chloride (see, for example, EP-A-306,984, page 2, lines 31-33), which has the following structure: N-benzyl-N-(C₈-C₁₈alkyl)-N,N-dimethylammonium chloride.

In another publication ophthalmic solutions are reviewed, which solutions comprise cyclodextrins and preservatives such as benzalkonium chloride (see Karl-Heinz Frömming and Josef Szejtli in Cyclodextrins in Pharmacy, 1994 Kluwer Academic Publishers, page 181, lines 10 - 21). The authors point out that the cyclodextrins may have a positive impact e.g. on the drug solubility or on the improvement of drug bioavailability. In contrast to this the same authors teach, that the cyclodextrins may also have a negative impact on such ophthalmic compositions, namely a decrease of the bioavailability of a drug, if the stability constant of the drug/cyclodextrin complex is very large, and in particular a loss of the preservative efficacy for certain preservatives such as e.g. benzalkonium chloride.

Therefore the problem to be solved consists of providing a preserved aqueous ophthalmic solution with good ocular tolerability, further comprising a pharmaceutically active compound having a high bioavailability when administered topically to the eye. In other words the problem to be solved consists of (1) maintaining the positive effects of both a cyclodextrin and of an ophthalmically acceptable quaternary ammonium salt within an ophthalmic composition designated for topical administration, and of (2) eliminating the negative impact of a cyclodextrin in particular with respect to a quaternary ammonium salt preservative.

Surprisingly it has been found, that a carefully balanced composition fulfills in fact all the criteria mentioned above for ophthalmic preparations. Accordingly a first aspect of the present invention is a preserved ophthalmic composition, comprising a cyclodextrin, a quaternary ammonium salt, an alkylene glycol and a pharmaceutically active compound.

In more detail, it has been found, that an alkylene glycol in accordance with the present invention exhibits a particular function which is different from ist known tonicity enhancing property. While an alkylene glycol may still act as tonicity and/or solubility enhancing agent, it has been found, that an alkylene glycol may enhance (or at least maintain) both the efficacy of a quaternary ammonium salt in its function as a preservative as well as the bioavailability enhancing efficacy of a cyclodextrin on a pharmaceutically active compound. In other words, all the effects known from a cyclodextrin itself on a pharmaceutically active compound or from a quaternary ammonium salt itself are typically enhanced in a synergistic fashion. An ophthalmic composition manufactured in accordance with the present invention will usually contain much lower concentrations of a preservative or of an ophthalmic drug as compared to a prior art composition. Accordingly such an ophthalmic composition will typically exhibit an excellent ophthalmic tolerability. In addition to this, an ophthalmic composition in accordance to the present invention will typically impart improved stability to a pharmaceutically active drug used.

Another aspect is a preserved ophthalmic composition, comprising a cyclodextrin, a quaternary ammonium salt, an alkylene glycol, a pharmaceutically active compound and a carrier.

The present invention also relates to a preserved ophthalmic composition, comprising a cyclodextrin, a quaternary ammonium salt, an alkylene glycol, a pharmaceutically active compound, a carrier and one or more of an excipient selected from the group consisting of buffers, solubilizers, complexing agents, stabilizers, preservatives different from quaternary ammonium salts, tonicity enhancing agents and fillers.

Another aspect is a preserved ophthalmic composition, comprising a cyclodextrin, a quaternary ammonium salt, an alkylene glycol, a pharmaceutically active compound, a carrier and a solubilizer.

Still another aspect is a preserved ophthalmic composition, comprising a cyclodextrin, a quaternary ammonium salt, an alkylene glycol, a pharmaceutically active compound, a carrier, a solubilizer and a complexing agent.

According to the invention an ophthalmic composition is advantageously applied topically to the eye, especially in the form of a solution, a suspension, an ointment, a gel or a solid insert. Such compositions comprise a pharmaceutically active ingredient, for example, in a range of from approximately 0.000001 to approximately 10.0% by weight, preferably from approximately 0.001 to approximately 1.0% by weight, or more preferably in the range of from approximately 0.01 to approximately 0.5% by weight and most preferably in the range of from 0.025 to 0.1% by weight. The dose of the active ingredient may depend on various factors, such as mode of administration, requirement, age and/or individual condition.

There are used for a corresponding ophthalmic composition other customary pharmaceutically acceptable excipients and additives known to the person skilled in the art, for example those of the type mentioned below, especially carriers, stabilizers, solubilizers, tonicity enhancing agents, buffer substances, preservatives different from quaternary ammonium salts, thickeners, complexing agents and other excipients. Examples of such additives and excipients can be found in U.S. Patents Nos. 5134124 and 4906613. Such compositions are prepared in a manner known per se, for example by mixing an active ingredient with the corresponding excipients and/or additives to form corresponding ophthalmic compositions.

Carriers used in accordance to the present invention are typically suitable for topical or general administration, and are for example water, mixtures of water and water-miscible solvents, such as C₁- to C₇-alkanols, vegetable oils or mineral oils comprising from 0.5 to 5% by weight hydroxyethylcellulose, ethyl oleate, carboxymethyl-cellulose, polyvinylpyrrolidone and other non-toxic water-soluble polymers for ophthalmic uses, such as, for example, cellulose derivatives, such as methylcellulose, alkali metal salts of carboxymethylcellulose, hydroxymethylcellulose, hydroxyethylcellulose, methylhydroxypropylcellulose and hydroxypropylcellulose, acrylates or methacrylates, such as salts of polyacrylic acid or ethyl acrylate, polyacrylamides, natural products, such as gelatin, alginates, pectins, tragacanth, karaya gum, xanthan gum, carrageenin, agar and acacia, starch derivatives, such as starch acetate and hydroxypropyl starch, and also other synthetic products, such as polyvinyl alcohol, polyvinylpyrrolidone, polyvinyl methyl ether, polyethylene oxide, preferably cross-linked polyacrylic acid, such as neutral Carbopol, or mixtures of those polymers. Preferred carriers are water, cellulose derivatives, such as methylcellulose, alkali metal salts of carboxymethylcellulose, hydroxymethylcellulose, hydroxyethylcellulose, methylhydroxypropylcellulose and hydroxypropylcellulose, neutral Carbopol, or mixtures thereof. The concentration of the carrier is, for example, from 1 to 100000 times the concentration of the active ingredient.

The solubilizers used for an ophthalmic composition of the present invention are, for example, tyloxapol, fatty acid glycerol polyethylene glycol esters, fatty acid polyethylene glycol esters, polyethylene glycols, glycerol ethers or mixtures of those compounds. A specific example of an especially preferred solubilizer is a reaction product of castor oil and ethylene oxide, for example the commercial products Cremophor EL® or Cremophor RH 40® . Reaction products of castor oil and ethylene oxide have proved to be particularly good solubilizers that are tolerated extremely well by the eye. Another preferred solubilizer is tyloxapol. The concentration used depends especially on the concentration of the active ingredient. The amount added is typically sufficient to solubilize the active ingredient. For example, the concentration of the solubilizer is from 0.1 to 5000 times the concentration of the active ingredient.

Buffers, tonicity enhancing agents and preservatives different from quaternary ammonium salts may be used in an ophthalmic composition of the present invention as well.

Examples of buffer substances are acetate, ascorbate, borate, hydrogen carbonate /carbonate, citrate, gluconate, lactate, phosphate, propionate and TRIS (tromethamine) buffers. Tromethamine and borate buffer are preferred buffers. The amount of buffer substance added is, for example, that necessary to ensure and maintain a physiologically tolerable pH range. The pH range is typically in the range of from 5 to 9, preferably from 6 to 8.5 and more preferably from 6.5 to 8.2.

Tonicity enhancing agents are, for example, ionic compounds, such as alkali metal or alkaline earth metal halides, such as, for example, CaCl₂, KBr, KCI, LiCI, Nal, NaBr or NaCI, or boric acid. Non-ionic tonicity enhancing agents are, for example, urea, glycerol, sorbitol, mannitol, propylene glycol, or dextrose. For example, sufficient tonicity enhancing agent is added to impart to the ready-for-use ophthalmic composition an osmolality of approximately from 50 to 1000 mOsmol, preferred from 100 to 400 mOsmol, more preferred from 200 to 400 mOsmol and even more preferred from 250 to 350 mOsmol.

Examples of preservatives different from quaternary ammonium salts are alkyl-mercury salts of thiosalicylic acid, such as, for example, thiomersal, phenylmercuric nitrate, phenylmercuric acetate or phenylmercuric borate, parabens, such as, for example, methylparaben or propylparaben, alcohols, such as, for example, chlorobutanol, benzyl alcohol or phenyl ethanol, guanidine derivatives, such as, for example, chlorohexidine or polyhexamethylene biguanide, or sorbic acid. Preferred preservatives are alkyl-mercury salts and parabens. Where appropriate, a sufficient amount of preservative is added to the ophthalmic composition to ensure protection against secondary contaminations during use caused by bacteria and fungi.

The ophthalmic compositions may comprise further non-toxic excipients, such as, for example, emulsifiers, wetting agents or fillers, such as, for example, the polyethylene glycols designated 200, 300, 400 and 600, or Carbowax designated 1000, 1500, 4000, 6000 and 10000. Other excipients that may be used if desired are listed below but they are not intended to limit in any way the scope of the possible excipients. They are especially complexing agents, such as disodium-EDTA or EDTA, antioxidants, such as ascorbic acid, acetylcysteine, cysteine, sodium hydrogen sulfite, butyl-hydroxyanisole, butylhydroxytoluene or alpha-tocopherol acetate; stabilizers, such thiourea, thiosorbitol, sodium dioctyl sulfosuccinate or monothioglycerol; or other excipients, such as, for example, lauric acid sorbitol ester, triethanol amine oleate or palmitic acid ester. Preferred exipients are complexing agents, such as disodium-EDTA. The amount and type of excipient added is in accordance with the particular requirements and is generally in the range of from approximately 0.0001 to approximately 90% by weight.

The pharmaceutically active compounds useful in accordance with the present invention may be selected from a wide variety of especially ophthalmically acceptable agents, including beneficial pharmaceutical agents, diagnostic agents, vitamins, nutrients, lubricants, and the like. The pharmaceutically active compounds may include, without limitation thereto, 3H-thymidine, acetylcholine chloride, acyclovir, adrenaline, amethocaine, aminocaproic acid, antazoline phosphate, arachidonic acid, atropine, benoxinate hydrochloride, betaxolol hydrochloride, bupivacaine, carbachol, carteolol, chloramphenicol, chlortetracycline hydrochloride, chymatrypsin, clonidine, cocaine, corynanthine, cromolyn sodium, cyclopentolate, demecarium bromide, dexamethasone, dibutoline, dichlorphenamide, diclofenac, dipivefrin hydrochloride, echodtiophate iodide, ephedrine, epinephrine bitartrate, erythromycin, ethambutol, etidocaine, eucatropine, fluoromethalone, fluorometholone, gentamycin sulfate, gramicidine, H-thymidine, homatropine hydrobromide, hyaluronic acid, hydrocortisone, idoxuridine, indomethacin, inositol triphosphate, inositol phosphates, isoflurophate, isosorbide, lachesine, levobunolol, levocabastine, lidocaine, lignocaine, medrysone, mepivacaine, methacholine, methazolamide, naphazoline hydrochloride, natamycin, neomycin sulfate, neostigmine, noradrenaline, ofloxacin, oxybuprocaine, oxymetazolin, oxyphenonium, pheniramine maleate, phenylephrine hydrochloride, phosphatidylinositol phosphates, physostigmine, pilocarpine hydrochloride, polymyxin B sulfates, prednisolone sodium phosphate, proparacaine hydrochloride, proxymethacaine, pyrilamine maleate, scopolamine hydrobromide, sorbinil, sulfacetamide, sulfisoxazole disolamine, tamoxifen, tetracaine hydrochloride, tetracycline, tetrahydrozoline hydrochloride, timolol maleate and hemihydrate, trifluridine, tropicamide, vidarabine, and salts and other ophthalmically acceptable salts, and mixtures thereof.

Preferred pharmaceutically active compounds are selected from the group of betaxolol hydrochloride, chloramphenicol, diclofenac, dipivefrin hydrochloride, ephedrine, epinephrine bitartrate, erythromycin, gentamycin sulfate, indomethacin, levobunolol, levocabastine, ofloxacin, pilocarpine hydrochloride, polymyxin B sulfates, prednisolone sodium phosphate, tetracycline and other ophthalmically acceptable salts thereof.

More preferred pharmaceutically active compounds are selected from the group of, betaxolol hydrochloride, chloramphenicol, diclofenac, dipivefrin hydrochloride, levobunolol, levocabastine, pilocarpine hydrochloride and other ophthalmically acceptable salts thereof.

A cyclodextrin as is referred to within the present invention is either an α-, β- or γ-cyclodextrin itself, a derivative thereof, e.g. a partially etherified derivative as e.g. a hydroxyalkyl ether or a mixture thereof. A random cyclodextrin does not automatically form an inclusion complex with any random pharmaceutical compound. Therefore the present invention relates preferably to such a cyclodextrin that meets the cavity needs of a pharmaceutical compound used. This may typically be accomplished by using either an α-, β- or γ-cyclodextrin itself or an appropriately substituted, α-, β- or γ- cyclodextrin or a mixture of the aforementioned cyclodextrins.

Appropriately substituted α-, β- or γ-cyclodextrins are e.g. alkylated, hydroxyalkylated, carboxyalkylated or alkyloxycarbonyl-alkylated derivatives. Other typical examples are carbohydrate derivatives of cyclodextrins such as mono- or diglycosyl-α-, β- or γ-cyclodextrin, mono- or dimaltosyl-α-, β- or γ- cyclodextrin or panosyl-cyclodextrin. Another parameter which describes the substitution pattern of a cyclodextrin is the degree of substitution (d.s.). The d.s. is another parameter in the determination of the cavity size of a cyclodextrin. A cyclodextrin is composed of several glucose units which have three free hydroxy groups per glucose. Accordingly the d.s. may vary from 0.125 up to 3. In the latter case all free (γ-cyclodextrin has 24) hydroxy groups may be substituted, while in the former case only 1 may be substituted. A minimal d.s. is preferred when γ-cyclodextrin is used as a solubilizer of a pharmaceutically active drug (see EP 197 571). A higher d.s. is e.g. preferred in technical applications and e.g. enzymes. In the latter instance, the higher d.s. causes that also those hydroxy groups are functionalized which are located in the cavity of the γ-cyclodextrin. Consequently the diameter of the cavity is decreased. By selecting the appropriate d.s. and the appropriate substituents the size of the cavity can be adapted in order to obtain the optimum space required for a certain molecule to appropriately fit into the cavity of the cyclodextrin. Preferably the d.s. may vary from 0.125 to 1.5 and more preferably from 0.125 to 0.5.

Preferred cyclodextrins are α-, β- and γ-cyclodextrin, derivatives and mixtures thereof.

Other preferred cyclodextrins are mono-, diglycosyl-β- cyclodextrin, mono- and dimaltosyl-β-cyclodextrin.

Strongly preferred cyclodextrins are hydroxypropyl-β-cyclodextrin, hydroxypropyl-γ-cyclodextrin, dimethyl-β- cyclodextrin and dimethyl-γ-cyclodextrin.

The amount of a cyclodextrin used in accordance with the present invention may range from 0.01 - 20 % by weight, preferably from 0.1 - 15 % by weight and more preferably from 1 - 10 % by weight.

The very small number of quaternary ammonium salts useful in eye medicaments for preservation is in contrast with the large number of known quaternary ammonium salts ("quats") which are employed for preservation in other fields. This is clearly restricted by the ocular tolerability of an addressed preservative. Accordingly in the present invention quaternary ammonium salts with excellent ocular tolerability are highly preferred.

The quaternary ammonium salts useful in accordance with the present invention may be selected from a wide variety of especially ophthalmically acceptable salts and are in particular selected from sepazonium chloride, cetyltrimethylammonium bromide (cetrimide), cetylpyridinium chloride, benzoxonium chloride, benzethonium chloride, domiphen bromide (Bradosol® ) and benzalkonium chloride

A preferred preservative useful in accordance with the present invention is benzalkonium chloride which has the following structure: N-benzyl-N-(C₈-C₁₈alkyl)-N,N-dimethylammonium chloride.

The amount of a quaternary ammonium salt used in accordance with the present invention may range from 0.00001 % by weight to 0.5 % by weight, preferably from 0.0001 % by weight to 0.1 % by weight and more preferably from 0.001 % weight to 0.05 % by weight.

Alkylene glycol useful in accordance with the present invention may be selected from a wide variety of alkylene glycols wherein alkylene has up to and including 18 C-atoms and is linear, cyclic or branched.

Examples of linear alkylene are methylene, 1,2-ethylene, 1,3-propylene, 1,4-butylene, 1,5-pentylene, 1,6-hexylene, 1,7-heptylene or 1,11-undecylene.

Examples of cyclic alkylene groups are 1,2-cis or trans cyclopropylene, 1,2- or 1,3- cis or trans cyclobutylene, the regio- and stereo-isomers of cyclopentylene, cyclohexylene or cycloheptylene.

Branched alkylene groups are for example 2-methyl-1,3-propylene, 2,2-dimethyl-1,4-butylene, 3-ethyl-2,4-butylene, 2, 3, 4-trimethyl-1,5-pentylene, 3-isopropy-1,6-hexylene or 3-methyl-1,7-heptylene.

A preferred alkylene glycol in accordance with the present invention is selected from an alkylene glycol with up to and including 7 C-atoms and is either linear, branched or cyclic. A more preferred alkylene glycol is selected from linear, branched and cyclic alkylene glycol having 3 to 7 C-atoms.

Highly preferred alkylene glycols are for example 1,2- or 1,3-propylene glycol, 1,2-, 1,3-, 2,3- or 1,4-butylene glycol or all isomers of pentylene, hexylene and heptylene glycol. Most preferred is propylene glycol.

The amount of an alkylene glycol used in accordance with the present invention may range from 0.01 - 20 % by weight, preferably from 0.1- 15 % by weight and more preferably from 1 - 10 % by weight.

Alkyl means throughout this invention an alkyl group having up to and including 18, more preferably 12 and even more preferably 7 C-atoms, and is either a linear or a branched alkyl group.

Examples for alkyl are methyl, ethyl, propyl, butyl, iso-propyl, t-butyl, neo-pentyl, octyl or dodecyl.

The term weight % used herein refers to the weight % of the total weight of an addressed composition or object.

The expression propylene glycol as used herein refers to 1,2-propylene glycol, unless specified differently.

Typical experimental procedures which illustrate the present invention, are described below.

| Example 1, eve drop formulations | | | | |
|---|---|---|---|---|
| diclofenac potassium | 1.00 mg | 0.5 mg | - | - |
| diclofenac sodium | - | - | 1.00 mg | 0.5 mg |
| tyloxapol USP | 1.00 mg | 1.00 mg | 1.00 mg | 1.00 mg |
| tromethamine | 1.00 mg | 1.00 mg | 1.00 mg | 1.00 mg |
| propylene glycol | 19.0 mg | 19.0 mg | 19.0 mg | 19.0 mg |
| hydroxypropyl-γ-cyclodextrin | 20.0 mg | 20.0 mg | 20.0 mg | 20.0 mg |
| disodium edetate | 1.00 mg | 1.00 mg | 1.00 mg | 1.00 mg |
| benzalkonium chloride | 0.05 mg | 0.05 mg | 0.05 mg | 0.05 mg |
| hydrochloric acid 1N | qs | qs | qs | qs |
| water for injections ad | 1.00 ml | 1.00 ml | 1.00 ml | 1.00 ml |
| pH | 7.96 | 7.98 | 7.96 | 7.98 |
| osmolality (mOsmol) | 305 | 303 | 305 | 303 |

| Example 2, eye gel formulations | | |
|---|---|---|
| diclofenac potassium | 1.00 mg | - |
| diclofenac sodium | - | 1.00 mg |
| tyloxapol USP | 1.00 mg | 1.00 mg |
| tromethamine | 6.50 mg | 6.50 mg |
| propylene glycol | 19.0 mg | 19.0 mg |
| hydroxypropyl-γ-cyclodextrin | 20.0 mg | 20.0 mg |
| disodium edetate | 1.00 mg | 1.00 mg |
| benzalkonium chloride | 0.05 mg | 0.05 mg |
| carbopol 980 | 3.50 mg | 3.50 mg |
| water for injections ad | 1.00 ml | 1.00 ml |
| pH | 8.00 | 8.00 |
| osmolality (mOsmol) | 308 | 308 |
| viscosity (mPa s) | 380 | 380 |

| Example 3, eye drop formulations | | | | |
|---|---|---|---|---|
| diclofenac potassium | 1.00 mg | 0.5 mg | - | - |
| diclofenac sodium | - | - | 1.00 mg | 0.5 mg |
| tromethamine | 1.00 mg | 1.00 mg | 1.00 mg | 1.00 mg |
| propylene glycol | 20.5 mg | 20.5 mg | 20.5 mg | 20.5 mg |
| hydroxypropyl-γ-cyclodextrin | 20.0 mg | 20.0 mg | 20.0 mg | 20.0 mg |
| disodium edetate | 1.00 mg | 1.00 mg | 1.00 mg | 1.00 mg |
| benzalkonium chloride | 0.06 mg | 0.06 mg | 0.06 mg | 0.06 mg |
| hydrochloric acid 1 N | qs | qs | qs | qs |
| water for injections ad | 1.00 ml | 1.00 ml | 1.00 ml | 1.00 ml |
| pH | 7.90 | 7.90 | 7.90 | 7.90 |
| osmolality (mOsmol) | 296 | 296 | 296 | 296 |

| Example 4; comparison of eye drop compositions with respect to their preservative efficacy according to the recommendations of the European Pharmacopoeia (Ph. Eur.). | | | | | |
|---|---|---|---|---|---|
| propylene glycol | 20.0 mg | 20.0 mg | | | 20.0 mg |
| tromethamine | 0.5 mg | 0.5 mg | 0.5 mg | 0.5 mg | |
| NaH₂PO₄ 2 H₂O | | | | | 0.6 mg |
| Na₂HPO₄ 12 H₂O | | | | | 6.0 mg |
| hydroxypropyl-β-cyclodextrin | 20.0 mg | 20.0 mg | 20.0 mg | | 20.0 mg |
| disodium edetate | 1.00 mg | 1.00 mg | 1.00 mg | 1.00 mg | 1.00 mg |
| benzalkonium chloride | 0.1 mg | | 0.1 mg | 0.1 mg | 0.1 mg |
| sodium chloride | | | 8.5 mg | 8.7 mg | |
| hydrochloric acid 10% | 0.6 mg | 0.2 mg | 0.3 mg | 0.1 mg | |
| water for injections ad | 1.00 ml | 1.00 ml | 1.00 ml | 1.00 ml | 1.00 ml |
| pH | 7.49 | 7.32 | 7.38 | 7.36 | 7.30 |
| osmolality (mOsmol) | 308 | 306 | 302 | 305 | 348 |
| preservative efficacy Ph. Eur. | B | r.n.m. | r.n.m. | A | B |

| | | | | | |
|---|---|---|---|---|---|
| A: Meets the criteria A recommended in the European Pharmacopoeia | | | | | |
| B: Meets the criteria B recommended in the European Pharmacopoeia | | | | | |
| r.n.m.: Recommendations of the European Pharmacopoeia not met. | | | | | |

The European Pharmacopoeia (Ph. Eur.) describes an efficacy test for antimicrobial preservation. Accordingly a preserved solution is inoculated with micro-organisms, characterized in that 10⁵ to 10⁶ micro-organisms are contained in one milliliter of the challenged preparation. The inoculum used does not exceed 1% of the total volume of said preparation. Five micro-organisms are used for the challenge, each separately namely, pseudomonas aeruginosa, staphylococcus aureus, candida albicans and aspergillus niger. The challenged solutions are kept at room temperature and protected from light. At regular time intervals samples are removed and the number of viable micro-organisms is determined either by plate count or by membrane filtration. For ophthalmic preparations the European Pharmacopoeia recommends criteria "A", which require e.g. that the bacterial micro-organisms are reduced by a factor of 1000, 24 hours after the challenge. Criteria "B" are still acceptable according to the recommendations of the European Pharmacopoeia, and require e.g. that the bacterial micro-organisms are reduced by a factor of 10, 24 hours after the challenge (for details refer to the European Pharmacopoeia, 1994). Accordingly, whenever the preservative efficacy recommendations of the European Pharmacopoeia are referred to herein, this relates to the 1994 version.

| Example 5, eye drop formulations containing a pharmaceutically active compound. Preservative efficacy is determined as in example 4. | | | | | |
|---|---|---|---|---|---|
| diclofenac potassium | 1.00 mg | 1.00 mg | 1.00 mg | 1.00 mg | 1.00 mg |
| propylene glycol | 20.0 mg | 20.0 mg | 20.0 mg | 20.0 mg | 20.0 mg |
| tromethamine | 0.5 mg | 0.5 mg | 0.5 mg | 0.5 mg | 0.5 mg |
| hydroxypropyl-β-cyclodextrin | 20.0 mg | | | | |
| hydroxypropyl-γ-cyclodextrin | | 20.0 mg | | | |
| α-cyclodextrin | | | 20.0 mg | | |
| β-cyclodextrin | | | | 15.0 mg | |
| γ-cyclodextrin | | | | | 20.0 mg |
| disodium edetate | 1.00 mg | 1.00 mg | 1.00 mg | 1.00 mg | 1.00 mg |
| benzalkonium chloride | 0.1 mg | 0.1 mg | 0.1 mg | 0.1 mg | 0.1 mg |
| hydrochloric acid 10 % | 0.4 mg | 0.5 mg | | 0.4 mg | 0.3 mg |
| water for injections ad | 1.00 ml | 1.00 ml | 1.00 ml | 1.00 ml | 1.00 ml |
| pH | 7.40 | 7.41 | 7.44 | 7.41 | 7.34 |
| osmolality (mOsmol) | 307 | 315 | 307 | 302 | 330 |
| preservative efficacy Ph. Eur | B | A | B | B | A |

| Example 6, eye drop formulation | |
|---|---|
| diclofenac sodium | 1.00 mg |
| propylene glycol | 20.0 mg |
| tromethamine | 0.5 mg |
| hydroxypropyl-β-cyclodextrin | 20.0 mg |
| disodium edetate | 1.00 mg |
| benzalkonium chloride | 0.1 mg |
| hydrochloric acid 10 % | 0.3 mg |
| water for injections ad | 1.00 ml |
| pH | 7.45 |
| osmolality (mOsmol) | 312 |
| preservative efficacy Ph. Eur | B |

| Example 7; eye drop formulations, not meeting the criteria as recommended in the European Pharmacopoeia. Preservative efficacy is determined as in example 4. | | | | | |
|---|---|---|---|---|---|
| diclofenac potassium | 1.00 mg | 1.00 mg | 1.00 mg | 1.00 mg | 1.00 mg |
| Cremophor EL | 20.0 mg | 20.0 mg | 20.0 mg | 20.0 mg | 20.0 mg |
| tromethamine | 0.5 mg | 0.5 mg | 0.5 mg | 0.5 mg | 0.5 mg |
| hydroxypropyl-β-cyclodextrin | 20.0 mg | | | | |
| hydroxypropyl-γ-cyclodextrin | | 20.0 mg | | | |
| α-cyclodextrin | | | 20.0 mg | | |
| β-cyclodextrin | | | | 15.0 mg | |
| γ-cyclodextrin | | | | | 20.0 mg |
| disodium edetate | 1.00 mg | 1.00 mg | 1.00 mg | 1.00 mg | 1.00 mg |
| benzalkonium chloride | 0.1 mg | 0.1 mg | 0.1 mg | 0.1 mg | 0.1 mg |
| sodium chloride | 8.00 mg | 8.00 mg | 8.00 mg | 8.00 mg | 8.00 mg |
| hydrochloric acid 10 % | 0.4 mg | 0.4 mg | | 0.3 mg | 0.2 mg |
| water for injections ad | 1.00 ml | 1.00 ml | 1.00 ml | 1.00 ml | 1.00 ml |
| pH | 7.44 | 7.44 | 7.49 | 7.47 | 7.44 |
| osmolality (mOsmol) | 308 | 309 | 301 | 311 | 296 |
| preservative efficacy Ph. Eur | r.n.m. | r.n.m. | r.n.m. | r.n.m. | r.n.m. |

## Claims

1. A preserved ophthalmic composition, comprising a cyclodextrin, a quaternary ammonium salt, an alkylene glycol and a pharmaceutically active compound.

2. A preserved ophthalmic composition according to claim 1, further comprising a carrier.

3. A preserved ophthalmic composition according to claim 1, further comprising one or more of an excipient selected from the group consisting of buffers, solubilizers, complexing agents, stabilizers, preservatives different from quaternary ammonium salts, tonicity enhancing agents and fillers.

4. A preserved ophthalmic composition according to claim 1, further comprising a carrier and a solubilizer.

5. A preserved ophthalmic composition according to claim 1, further comprising a carrier, a solubilizer and a complexing agent.

6. A preserved ophthalmic composition according to claims 1 to 5, wherein the composition meets the preservative efficacy recommendations of the European Pharmacopoeia (1994 version).

7. A preserved ophthalmic composition according to claims 1 to 5, wherein the quaternary ammonium salt is selected from sepazonium chloride, cetyltrimethylammonium bromide (cetrimide), cetylpyridinium chloride, benzoxonium chloride, benzethonium chloride, domiphen bromide (Bradosol® ) and benzalkonium chloride.

8. A preserved ophthalmic composition according to claims 1 to 5, wherein the cyclodextrin is selected from α-, β- and γ-cyclodextrin, derivatives and mixtures thereof.

9. A composition according to claim 8, wherein the cyclodextrin is selected from mono-, diglycosyl-β- cyclodextrin, mono-, dimaltosyl-β-cyclodextrin, hydroxypropyl-β-cyclodextrin, hydroxypropyl-γ-cyclodextrin, dimethyl-β- cyclodextrin and dimethyl-γ-cyclodextrin.

10. A preserved ophthalmic composition according to claims 1 to 5, wherein the alkylene glykol is selected from linear, branched and cyclic alkylene glycol having up to 7 C-atoms.

11. A composition according to claim 10, wherein the alkylene glykol is selected from linear, branched and cyclic alkylene glycol having 3 to 7 C-atoms.

12. A preserved ophthalmic composition according to claim 2, wherein the carrier is selected from water, cellulose derivatives, such as methylcellulose, alkali metal salts of carboxymethylcellulose, hydroxymethylcellulose, hydroxyethylcellulose, methylhydroxypropylcellulose and hydroxypropylcellulose, neutral Carbopol and mixtures thereof.

13. A preserved ophthalmic composition according to claims 3 or 4, wherein the solubilizer is selected from tyloxapol, fatty acid glycerol polyethylene glycol esters, fatty acid polyethylene glycol esters, polyethylene glycols, glycerol ethers and mixtures of those compounds.

14. A preserved ophthalmic composition according to claims 3 or 5, wherein the complexing agent is EDTA or disodium EDTA.

15. A preserved ophthalmic composition according to claim 1, wherein the pharmaceutically active compound is selected from the group of betaxolol hydrochloride, chloramphenicol, diclofenac, dipivefrin hydrochloride, levobunolol, levocabastine, pilocarpine hydrochloride and salts or other ophthalmically acceptable salts thereof.

## Patentansprüche

1. Konservierte ophthalmische Zusammensetzung, die ein Cyclodextrin, ein quarternäres Ammoniumsalz, ein Alkylenglykol und eine pharmazeutisch wirksame Verbindung umfasst.

2. Konservierte ophthalmische Zusammensetzung nach Anspruch 1, die zusätzlich einen Träger umfasst.

3. Konservierte ophthalmische Zusammensetzung nach Anspruch 1, die einen oder mehrere Zuschlagstoffe umfasst, ausgewählt aus Puffern, Lösevermittlern, Komplexbildnern, Stabilisatoren, anderen Konservierungsmitteln als quarternäre Ammoniumsalze, tonizitätserhöhenden Mitteln und Füllstoffen.

4. Konservierte ophthalmische Zusammensetzung nach Anspruch 1, die zusätzlich einen Träger und einen Lösevermittler umfasst.

5. Konservierte ophthalmische Zusammensetzung nach Anspruch 1, die zusätzlich einen Träger, einen Lösevermittler und einen Komplexbildner umfasst.

6. Konservierte ophthalmische Zusammensetzung nach Anspruch 1 bis 5, deren Zusammensetzung den Empfehlungen über die Konservierungswirkung der Europäischen Pharmacopoeia (Version 1994) entspricht.

7. Konservierte ophthalmische Zusammensetzung nach Anspruch 1 bis 5, in der das quarternäre Ammoniumsalz aus Sepazoniumchlorid, Cetyltrimethylammoniumbromid, (Cetrimid), Cetylpyridiniumchlorid, Benzoxoniumchlorid, Benzethoniumchlorid, Domiphenbromid (Bradosol® ) und Benzalkoniumchlorid ausgewählt ist.

8. Konservierte ophthalmische Zusammensetzung nach Anspruch 1 bis 5, in der das Cyclodextrin aus α-, β- und γ-Cyclodextrin, Derivaten und Gemischen davon, ausgewählt ist.

9. Zusammensetzung nach Anspruch 8, in der das Cyclodextrin aus Mono-, Diglycosyl-β-cyclodextrin, Mono-, Dimaltosyl-β-cyclodextrin, Hydroxypropyl-β-cyclodextrin, Hydroxypropyl-γ-cylodextrin, Dimethyl-β-cyclodextrin und Dimethyl-y-cyclodextrin ausgewählt ist.

10. Konservierte ophthalmische Zusammensetzung nach Anspruch 1 bis 5, in der das Alkylenglykol aus linearen, verzweigten und zyklischen Alkylenglykolen mit bis zu 7 C-Atomen ausgewählt ist.

11. Zusammensetzung nach Anspruch 10, in der das Alkylenglykol aus linearen, verzweigten und zyklischen Alkylenglykolen mit 3 bis 7 C-Atomen ausgewählt ist.

12. Konservierte ophthalmische Zusammensetzung nach Anspruch 2, in welcher der Träger aus Wasser, Cellulosederivaten, wie Methylcellulose; Alkalimetallsalzen von Carboxymethylcellulose; Hydroxymethylcellulose, Hydroxyethylcellulose, Methylhydroxypropylcellulose und Hydroxypropylcellulose; neutralem Carbopol und Gemischen dieser Verbindungen ausgewählt ist.

13. Konservierte ophthalmische Zusammensetzung nach Anspruch 3 oder 4, in welcher der Lösevermittler aus Tyloxapol, Fettsäure-Glycerin-Polyethylenglykolester, Fettsäure-Polyethylengylkolester, Polyethylenglykolen, Glycerinethern und Gemischen dieser Verbindungen ausgewählt ist.

14. Konservierte ophthalmische Zusammensetzung nach Anspruch 3 oder 5, in welcher der Komplexbildner EDTA oder Dinatrium-EDTA ist.

15. Konservierte ophthalmische Zusammensetzung nach Anspruch 1, in der die pharmazeutisch wirksame Verbindung aus Betaxololhydrochlorid, Chloramphenicol, Diclofenac, Dipivefrinhydrochlorid, Levobunolol, Levocabastin, Pilocarpinhydrochlorid und Salzen oder anderen ophthalmisch verträglichen Salzen davon ausgewählt ist.

## Revendications

1. Une composition ophtalmique conservée, comprenant une cyclodextrine, un sel d'ammonium quaternaire, un alkylèneglycol et un composé pharmaceutiquement actif.

2. Une composition ophtalmique conservée selon la revendication 1, comprenant en outre un véhicule.

3. Une composition ophtalmique conservée selon la revendication 1, comprenant en outre un ou plusieurs excipients choisis parmi le groupe comprenant des tampons, des solubilisants, des agents complexants, des stabilisants, des conservateurs différents des sels d'ammonium quaternaires, des agents augmentant la tonicité et des charges.

4. Une composition ophtalmique conservée selon la revendication 1, comprenant en outre un véhicule et un solubilisant.

5. Une composition ophtalmique conservée selon la revendication 1, comprenant en outre un véhicule, un solubilisant et un agent complexant.

6. Une composition ophtalmique conservée selon les revendications 1 à 5, où la composition est conforme aux recommandations d'efficacité de conservation de la Pharmacopée européenne (version de 1994).

7. Une composition ophtalmique conservée selon les revendications 1 à 5, où le sel d'ammonium quaternaire est choisi parmi le chlorure de sépazonium, le bromure de cétyltriméthylammonium (cétrimide), le chlorure de cétylpyridinium, le chlorure de benzoxonium, le chlorure de benzéthonium, le bromure de domiphène (Bradosol®) et le chlorure de benzalkonium.

8. Une composition ophtalmique conservée selon les revendications 1 à 5, où la cyclodextrine est choisie parmi l'α-, la β- et la γ-cyclodextrine, leurs dérivés et leurs mélanges.

9. Une composition selon la revendication 8, où la cyclodextrine est choisie parmi la mono-, diglycosyl-β-cyclodextrine, la mono-, dimaltosyl-β-cyclodextrine, l'hydroxypropyl-β-cyclodextrine, l'hydroxypropyl-γ-cyclodextrine, la diméthyl-β-cyclodextrine et la diméthyl-γ-cyclodextrine.

10. Une composition ophtalmique conservée selon les revendications 1 à 5, où l'alkylèneglycol est choisi parmi un alkylèneglycol linéaire, ramifié et cyclique ayant jusqu'à 7 atomes de carbone.

11. Une composition selon la revendication 10, où l'alkylèneglycol est choisi parmi un alkylèneglycol linéaire, ramifié et cyclique ayant de 3 à 7 atomes de carbone.

12. Une composition ophtalmique conservée selon la revendication 2, où le véhicule est choisi parmi l'eau, les dérivés de la cellulose tels que la méthylcellulose, les sels de métaux alcalins de la carboxyméthylcellulose, de l'hydroxyméthylcellulose, de l'hydroxyéthylcellulose, de la méthylhydroxypropylcellulose et de l'hydroxypropylcellulose, le Carbopol neutre et leurs mélanges.

13. Une composition ophtalmique conservée selon les revendications 3 ou 4, où le solubilisant est choisi parmi le tyloxapol, les esters d'acides gras du glycérol et du polyéthylèneglycol, les esters d'acides gras du polyéthylèneglycol, les polyéthylèneglycols, les éthers du glycérol et les mélanges de ces composés.

14. Une composition ophtalmique conservée selon les revendications 3 ou 5, où l'agent complexant est l'EDTA ou l'EDTA disodique.

15. Une composition ophtalmique conservée selon la revendication 1, où le composé pharmaceutiquement actif est choisi parmi le groupe du chlorhydrate de bétaxolol, le chloramphénicol, le diclofénac, le chlorhydrate de dipivéfrine, le lévobunolol, la lévocabastine, le chlorhydrate de pilocarpine et leurs sels ou d'autres de leurs sels ophtalmiquement acceptables.
